(19) Europäisches Patentamt
European Patent Office
Office européen des brevets

(11) **EP 4 635 404 A1**

(12) **EUROPEAN PATENT APPLICATION**

(43) Date of publication:
**22.10.2025 Bulletin 2025/43**

(21) Application number: **24472007.4**

(22) Date of filing: **15.04.2024**

(51) International Patent Classification (IPC):
**A61B 5/00** $^{(2006.01)}$  **A61B 5/06** $^{(2006.01)}$
**G01B 7/004** $^{(2006.01)}$  **G06F 3/01** $^{(2006.01)}$

(52) Cooperative Patent Classification (CPC):
**A61B 5/6806; G01B 7/004; G06F 3/014;**
A61B 5/065

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Madesign OOD**
**1612 Sofia (BG)**

(72) Inventors:
• **Mahdaoui, Sabri**
**Sofia (BG)**
• **Stavrev, Valentin**
**Sofia (BG)**

(74) Representative: **Benatov, Samuil Gabriel**
**Dr. EMIL BENATOV & PARTNERS**
**6, Asen Peykov Str.**
**1113 Sofia (BG)**

(54) **ACTIVE MAGNETIC ORIENTATION AND POSITION MEASUREMENT SYSTEM AND METHOD FOR ACTIVE MAGNETIC ORIENTATION AND POSITION MEASUREMENT**

(57) The invention relates to an active magnetic orientation and position measurement system and method, which utilize solid-state magnetic sensors (210) and local altering pulsed magnetic sources.

Fig. 1

## Description

## FIELD OF APPLICATION

The active magnetic orientation and position measurement system and the method for active magnetic orientation and position measurement are applicable in various technical fields using means for orientation and position measurement, characterized by the use of electric or magnetic techniques, and radio direction-finding, used in motion capture wearable systems.

## PRIOR ART

Various wearable tracking systems are known in the state of the art. Small wearable inertial/magnetic sensors have become increasingly popular due to their availability and low cost as a consequence of their application in mobile devices. They are adapted for many applications, including measurements of the movement and orientation of the human body or body segments. The process is known as 'Motion Capture.' Several manufacturers have integrated magnetometers into inertial measurement units (IMUs) to construct magnetic and inertial measurement units (MIMU) that provide higher degrees of freedom and more accurate absolute orientation. The inherent offset and noise have serious effects on the outputs of low-cost gyroscopes and accelerometers of IMU, resulting in drift over time. External references are needed to correct the draft periodically. One reference is the gravity vector obtained from accelerometer data. The second one is usually the Geomagnetic field. In order to measure 3D orientation, it is necessary to convert sensor data into information that represents a 3D orientation, such as Euler angles or quaternions. This conversion is also known as sensor fusion, which is the combination of raw data from disparate sensors such as accelerometer, gyroscope, and magnetometer, such that the combined result is more accurate, more complete, and more reliable. This is why multiple sensors are used together to measure 3D orientation. Generally, a Kalman filter is used. In addition, quaternion-based and gradient descent-based methods have been developed for this. Other filtering technologies, such as low-pass and high-pass filters, are also often used to remove noise or drift from sensor data. Magnetometers are also subject to interference from surrounding ferromagnetic materials. This may also lead to severe drift and distortion in 3D measurements and estimations. In contrast to ubiquitous gravity, the magnetic field of the earth is subject to disturbance and distortion. That fact makes problematic use of absolute orientation MIMU indoors and in an industrial environment.

The Electromagnetic and tracking systems are much older than MIMU motion capture systems. They are still the primary choice in medical applications despite their high price, size, and power consumption. The operation of six-degree-of-freedom electromagnetic trackers is based on the spatial properties of the electromagnetic fields generated by three orthogonal coils. The coils are excited by AC current in most cases. Another set of coils is used for receiving the signal. The received signal further is amplified and processed by analogue and/or digital means. The magnetic field around transmitter coils is possible to be calculated or mapped. By measuring the direction and strength of the field generated by transmitters' coils, it is possible to retrieve the orientation and position of the receiver relative to the transmitter. The algorithms to do such reverse transformation are subject to multiple patents, such as US5307072. The major limitation is rooted in fundamental physical law - the magnetic field strength varies as an inverse cube with the distance to the source. Experimental investigations confirm that the inverse cube law has major impact on the performance of systems relying on the physics of electromagnetic fields. Furthermore, errors due to external electrical fields and nearby metal increase as the fourth power of transmitter-receiver separation.

Sensor fusion of inertial and magnetic sensors is a common technic for drift removal and providing absolute orientation in commercially available devices, the so-called 9 DoF IMU. They used an external reference, the earth magnetic field. Using the geomagnetic field as a reference has a significant drawback - the system is unreliable indoor and in an industrial environment.

## SUMMARY OF THE INVENTION

The object of the invention is to create a magnetic orientation and tracking system, that solves the drawbacks of the state of the art.

This object is achieved by creating an active magnetic orientation and position measurement system comprising a switchable reference module including at least one solid-state magnetic sensor (also called receiver) and at least one magnetic transmitter, which reference module is provided with means for connecting to a power source (also called power unit), such as DC current source, and a processor configured to send and receive data and commands for signal continuity from the reference module. The at least one solid-state magnetic sensor is configured to receive signal from the at least one magnetic transmitter, which comprises at least one driver circuit and at least one transmitter coil for generation excitation current in the form of altering DC pulses, applied to a pulse magnetic field.

In a preferred embodiment, the at least one driver circuit is a H-bridge driver.

The at least one transmitter coil is configured to generate switching in opposite direction magnetic vectors forming a magnetic field, and in that the generated magnetic field is steady in switched-on position of the at least one transmitter coil, and in that the processor is configured to determine current position of the at least one transmitter relative to the position of the at least one solid-state magnetic sensor, based on two subsequent measurements of said magnetic field, wherein for each pair of two subsequent measurements the first measurement is used as local reference for the second.

The magnetic position and orientation systems are generally of two types: AC (alternated current, periodic usually sinusoidal) and DC (direct current). The DC systems use a magnetic field that becomes steady during the measurement. The process presented in this invention works by altering DC pulses detached in time. It is not mandatory these pulses to be periodic.

The reason for such arrangement is primarily the sensor used in the system. The monolithic magnetometers measure the DC magnetic field. A basic problem of monolithic magnetic sensors is the Zero Offset. By the procedure described in this invention, the zero offset is compensated, because the magnetic vectors in opposite directions, with no additional calibration usually taking place when monolithic (solid-state) sensors are used.

Using a transmitter signal with DC which switches in opposite direction of coils in two consecutive measurements allows to eliminate most of disturbance and noise, because the difference between the two measurements is calculated and thus the disturbances and noise is separated. Present invention uses a steady magnetic field after the transmitter coil is switched on.

The present invention uses a mapping of the actual field around coils instead of an analytical or dipole approximation in order to optimize the efficiency of the coils.

In a preferred embodiment of the invention, the reference module is integrated with or connected to a micromechanical gyroscope and an accelerometer to measure additionally orientation and angular velocity, and acceleration of motion.

In a preferred embodiment of the invention, the at least one solid-state magnetic sensor is a 3-axis solid-state magnetic sensor. It is used for 3-dimensional position and orientation.

In a preferred embodiment of the invention, at least one inertial sensor is integrated with the at least one solid-state magnetic sensor, in order to obtain fusion of orientation data.

In a preferred embodiment of the invention, it further comprises a Kalman filter for blending the available data from the inertial sensors and magnetic system efficiently.

In one embodiment of the invention, it includes a chain of solid-state magnetic sensors and magnetic transmitters for noninvasive monitoring of the dynamic shape of a human spine, where solid-state magnetic sensors and magnetic transmitters are connected together by serial bus. The bigger the distance of the measured object is, the stronger sensors are required or more sensors in a chain.

In another embodiment of the invention, one magnetic transmitter and five solid-state magnetic sensors are included. They are connected together by serial bus and are integrated into a glove-like wearable. The magnetic transmitter is situated on a palm of the glove-like wearable, and the five solid-state magnetic sensors are situated on the intermediate phalange of all five fingers of the glove-like wearable. The system comprises further a sixth solid-state magnetic sensor which is situated outside the glove for tracking the movement of a human wrist.

An additional object of the invention is to create an optimized method for active magnetic orientation and position measurement.

This object is met by creating a method for active magnetic orientation and position measurement with an active magnetic orientation and position measurement system, comprising the steps of

- providing DC current as a result of a command from a processor to at least one transmitter coil from a reference module in a first direction;
- after a delay programmed in the processor, cutting out transient processes, the processor receives data from at least one solid-state magnetic sensor from the reference module regarding the orientation and position in the first direction;
- providing DC current as a result of a command from a processor to the at least one transmitter coil from the reference module in a second direction opposite to the first one;

- after delay programmed in the processor, cutting out transient processes, the processor receives data from the at least one solid-state magnetic sensor regarding the orientation and position in the second direction opposite to the first one, and determining a current position of the at least one solid-state magnetic sensor;
- generating new DC current pulses in the first and second direction in the at least one transmitter coil for a subsequent measurement of orientation and position;
- using the first measured values from the generated magnetic field as a local reference to determine a new current orientation and position of the at least one solid-state magnetic sensor.

In a preferred embodiment of the invention, the reference module includes more than one transmitter and solid-state magnetic sensors, which orientations and positions are also measured in subsequent periods based on the measurement of the generated magnetic vectors in two opposite directions.

The invention is independent of the Earth's magnetic field and uses local reference, which increases the efficiency of the system and the method.

The switching of magnetic vectors in opposite directions and measurement and subtraction of measured values is the core of the presented implementation. The main benefits of the presented system and method are:

a. Remove the constant or low-changing component of the external interference because of the use of vectors in opposite directions.

b. Compensate the sensor offset because of the use of vectors in opposite directions.

c. Reduce the additive random noise.

d. Compensating the noise generated by magnetic pulses in surrounding metals and preventing the magnetization because the impulses are in different directions, if something is magnetized, it allows to be later demagnetized.

## DESCRIPTION OF THE FIGURES

Fig. 1 shows a scheme of transmitting of magnetic pulses from source, with represented: coil drivers, associated push-pull H-bridge drivers, processor unit, and solid-state sensor, wherein a processor (MCU) (also called microcontroller) and power unit control and power the drivers, read the monolithic sensors and calculate orientation and position.

Fig. 2 shows a lock-in amplifier principle of operation, wherein the test signal is modulated (chopped), and the solid-state magnetic sensor is synchronously demodulated.

Fig. 3 shows timing diagrams, wherein the alternating DC pulses and the sequence of measurements is illustrated, and the cut-out of transient processes during the rise of magnetic field pulses are shown.

Fig. 4 shows a mutual orientation and kinematic chain, wherein any node of the chain serves as a reference for the next and vice versa.

Fig. 5 shows a transmitter (TX) - receiver (RX) chain in an application as kinematic chain in a preferred embodiment of spine and torso monitoring system.

Fig. 6 shows one embodiment of the invention, namely motion-tracking gloves, wherein a single transmitter provides a local reference for six magnetic sensors.

Fig. 7 shows a sensor fusion, wherein the result of magnetic measurements is not used in the fusion scheme directly, but first, the position and orientation are calculated from magnetic measurement data.

Fig. 8 shows a channel internalising scheme, wherein the magnetic pulses of different channels are shifted.

## EXEMPLARY EMBODIMENT OF THE INVENTION

The invention is related to a magnetic orientation and tracking system and method.

The system consists of at least one pair of a transmitter that generates magnetic pulses and a solid-state magnetic sensor that takes measurements of the magnetic field in the respective moment, and a processor and power unit. Up to three orthogonal magnetic sources may be used in one transmitter. The number of sources depends on the degree of freedom needed for the particular application. For example, for tracking the knee joint movement, a single angle measurement is sufficient due to anatomical constraints. In contrast, in the head movement tracking relative to the torso, six degrees of freedom are needed.

Different objects can be tracked to measure their position or orientation.

The position or orientation of a three-axis magnetic source can be tracked using a corresponding three-axis magnetic sensor that detects variations in a magnetic field produced by the switchable magnetic source. For 3- dimensional position and orientation, 3 orthogonal magnetic sources are needed. For lower dimensionality (e.g., restrained movement as in cylindrical joint), one or two magnetic sources will be enough.

The magnetic field source coils and 3-axis solid-state sensor derived from off-the-shelf suppliers in a magnetic tracker are integrated with driving and processing circuitry to provide a complete up to six-degree-of-freedom tracking system.

The magnetic source can implement coils that generate magnetic fields in response to electric currents applied to the coils. A three-axis magnetic source can be operated with pulsed direct current (DC) in which currents are applied to each of the three orthogonal coils in sequence (time multiplexing). This operational mode can be referred to as a DC-pulsed time-multiplexed mode in which three orthogonal coils in the three-axis magnetic sensor generate electric currents in response to the magnetic fields provided by the three-axis magnetic source in the multiplexed time intervals.

The invention includes driver circuit 201, 203, 205, that is designed to control the operation of another electronic device at low power levels, for example, it is able to output specific voltage pulses to a coil. Embodiments of driver circuits can be H-bridge driver, or alternatively a function generator with power amplifier, or MOSFET or IGBT Push-Pull Configuration.

Variants of H-bridge driver can be bipolar transistor H-bridge, Half-Bridge Drivers, or other suitable H-bridge drivers, known from the state of the art.

So, for all embodiments mentioned in the text, using H-bridge drivers, alternative driver circuits, as mentioned above, can be used to replace the H-bridge drivers.

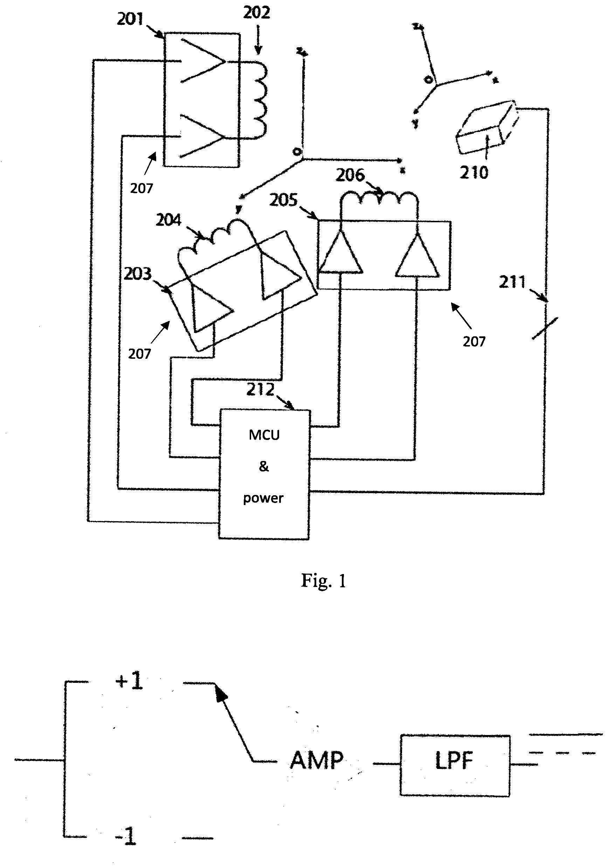

In fig.1 is shown an apparatus, comprising switchable alternate source of magnetic field: a transmitter 207 comprised of coils 202, 204, 206 and associated H-bridge drivers 201, 203, 205; off-the-shelve (monolithic) solid-state magnetic sensors optionally are integrated or mechanically coupled with MEMS gyroscope and accelerometer; unit 212 including processor (MCU) and means for connecting to a power source.

Magnetic coils are utilized as a source of a switchable magnetic field. The said coils may have a ferrite core in order to achieve a reasonable magnetic field strength at low current and minimal physical coil size. The coils are driven by H-bridge drivers to alternate the current through the coil and this way to generate a reversible magnetic pulse. The reverse of the magnetic field during the measurement cycle is a distinguishing feature of the operational mode of the presented invention. The control inputs of the drivers may be connected directly to the microcontroller ports or to use a serial interface 211 such as I$^2$C or SPI or another suitable serial interface 211 known from the state of the art.

An off-the-shelf monolithic magnetic sensor (often referred to as an electronic compass) is applied as the receiver. Several types of such sensors are available depending on the physical principles they work with. Hall effect, magnetoresistive, and tunnel magnetoresistive are most popular. In the preferred embodiment, we use a monolithic complete 3-axis Anisotropic MagnetoResistive (AMR) magnetic sensor with on-chip signal processing and an integrated digital bus. The magnetoresistive sensor uses the fact that the electrical resistance in a ferromagnetic thin film alloy is changed through an external magnetic field. These sensors are exceptionally small, and due to their special material, they are robust and consume very little energy. Hall effect sensors are based on a different physical principle - the Hall effect. Generally, they provide lower sensitivity but a higher sampling rate. The tunnel magnetoresistive (TMR) sensor is the newest development in the field and provides the highest sensitivity. The update rate (the sampling frequency, bandwidth) of most of the available monolithic magnetic sensors with a sensitivity suitable for the presented system is 100 Hz to 1 kHz.

The monolithic magnetic sensors are rather complex devices (monolithic integrated circuit), including a magnetic transducer, compensation circuit, signal processing, and digital interface. The transducer is doing a sensing in the receiver that senses the signal and difference. The transducer transforms magnetic field into voltage. The operational mode typically includes reading request, analogue reading, analogue-to-digital convention, normalization and compensation of the data. All that operations are done internally by the embedded processor. Only a few parameters guiding this process, such as range and calibration options, are accessible for change via the digital interface. At the end of the measuring cycle, the result of measurement is transmitted through the serial interface. The output is a digital presentation of the magnetic field measurement in a particular moment referred to as a measuring period. That fact prevents the direct application of signal processing techniques such as Synchronous demodulation. Instead, the present method manipulates the output data implementing the synchronous demodulation scheme in software described further.

The common technique of suppression of narrow band internees by synchronization of the sampling rate with the frequency of the interference (noise) is applicable to the described system.

The period when the solid-state magnetic sensor made the actual measurement is a relatively small portion of the measurement cycle. This is typically the time interval from hungered microseconds to a few milliseconds. The exact timing of the measurement process may be retrieved from the manufacturer documentation or measured directly. Generally, the measurement period lasts less than 100 microseconds and is about 1/10 of the output data update rate. The fact the measuring period is relatively short is used in the present invention to shorten as possible the duration of the transmitter's magnetic pulse and, this way, to reduce the power consumption.

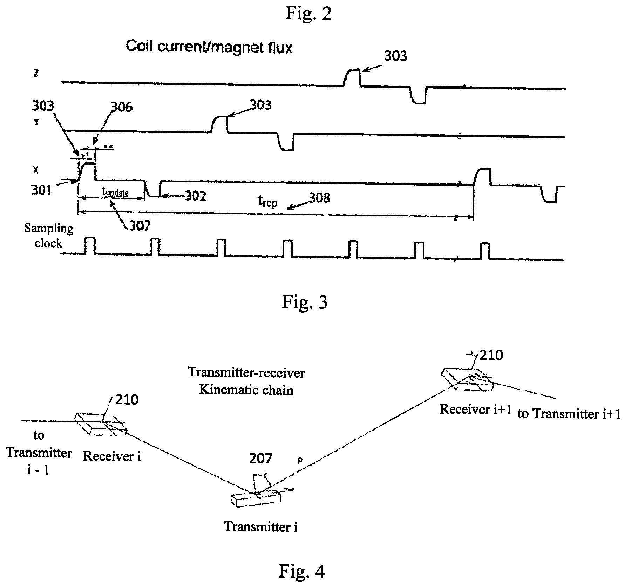

According to the invention, as shown in fig. 1 and 3, the transmitter side comprises:

    i. A source of alternated magnetic pulses - at least one coil 202, 204, 206;
    ii. Driver(s) 201 providing an alternating current through these coils 202, 204, 206;
    iii. Optional IMU mechanically coupled with said transmission coils 202, 204, 206 providing a first reference frame.

According to the invention, the receiver side comprises:

    i. Solid-state magnetic sensor 210;
    ii. Optional IMU integrated with said IMU or mechanically coupled with said magnetic sensor and providing a second reference frame.

Processor and power unit are connected to transmitter side and receiver side and provide power, control signals, and reading and processing data from sensors.

The mode of operation of the transmitter-receiver pair includes two major steps:

    a. Position and orientation measurement by the magnetic coils and sensors;
    b. Optional fusion of the result of said measurement with IMU data.

Position and orientation measurement by the magnetic coils and sensors consist of the following steps:

    a. At start time 301, generation of a magnetic pulse by the first coil in the transmitter side in the first direction by activating the associated driver 201 by the processor unit command;
    b. Reading the magnetic sensor after a programmed delay 305 in order to cut out the transition processes that starts at time 301 in the coil and eventually the eddy current in nearby metals;
    c. After a delay $t_{update}$ 307, repetition of steps a) and b) by inverted magnetic pulse 302;

d. Subtraction of sensor readings obtained after emission of alternate pulses 302, 303. This step removes the constant magnetic component, for example, Earth's magnetic field, and reduces additive noise;

e. Optional repetition of steps a) to c) and filtering the obtained sequence to improve the signal to noise ratio;

f. Calculation of position and orientation of the second reference frame (magnetic sensor) in respect to the first reference frame (transmitter coil) from the measured magnetic vector. The magnitude of this vector depends on the distance between the coil and the sensor and its orientation. The six parameters of position and orientation in 3D space can be calculated from nine measurements by the 3-axis magnetometer of the field generated by three orthogonal coils.

A separation of channels is needed when more than one transmitter coil is used in the system. In AC (alternating current) magnetic tracking systems, frequency separation is the most common. In the presented system DC (direct current) pulses must be separated in time by a technique known as time multiplexing. The transmitter coils are switched in a sequence that ensures that within the measuring period of the targeted sensor(s), the field generated by any but one coil is zero.

The measurement period of the magnetic sensor is synchronised with the steady state 306 of the current pulse in the transmitter coil. It could be regarded as the equivalency of the sampling.

Transient processes such as rising time, overshoot oscillations, etc., are cut out by a programmed delay of sampling after the front of the pulse current in the coil. The magnetic field is kept constant (in steady state) 306 during the measurement for a certain time window.

The measurement period is a small portion of the whole measurement cycle which typically includes also analogue to digital conversion, signal processing, and data transfer. This fact allows the direct current in coils to be switched on in a low duty cycle and this way to optimize the power consumption.

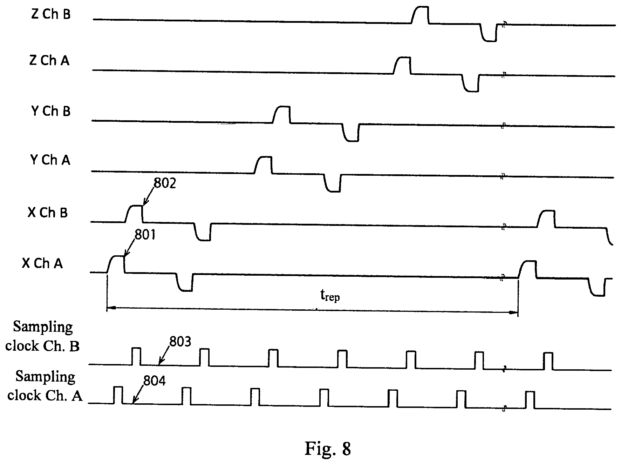

The problem of channel separation arose when more than one transmitter is used in the presented system. The low duty cycle allows the magnetic pulses of more than one transmitter to be interlaced. In this case the overall measurement cycle has a period of $t_{rep}$ 308 when all the coils are activated one after the other. The operational mode of multi-transmitter - the multi-receiver arrangement is described in the preferred embodiment. Generally, the idea is to interleave the transmitters pulses of different channels, as is shown in Fig. 8.

Process applied to drift-free inertial measurements

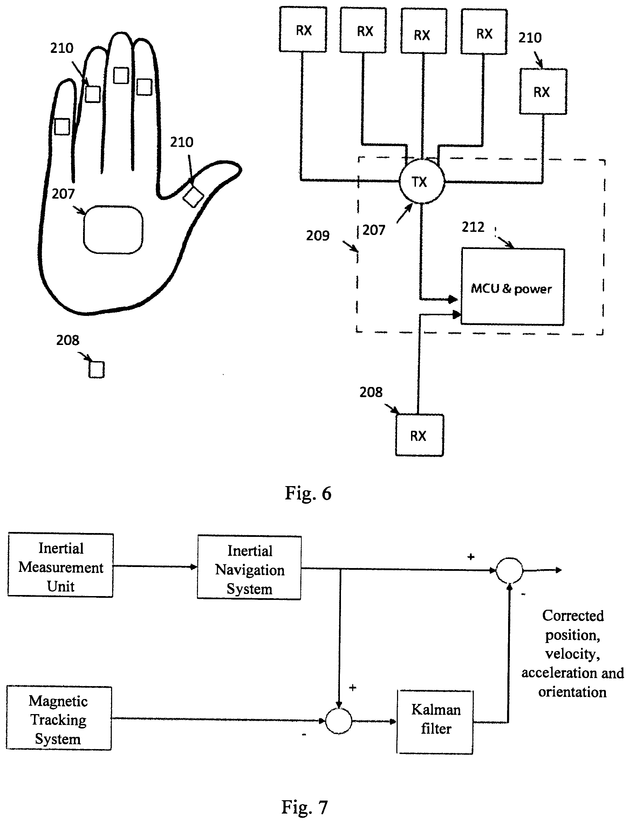

In another aspect of the present invention, the described active magnetic position and orientation method are also used for drift-free reference of IMU and fusion with IMU data. The block diagram of sensor fusion is shown in Fig 7. The idea is to combine the fact reaction and output data rate of IMU and drift-free measurement of the described active magnetic position and orientation system. The combined 'fused' system uses a modified fusion scheme of sensor data. A specific difference of fusion of local magnetic reference with inertial sensors data is that reference is no longer inertial due to the reference frame being a local one and, in general, is not inertial. The use of magnetic reference alongside IMU provides a high update rate, and an active magnetic system provides updates of the position and orientation drift correction at a lower update rate. The fusion of inertial and magnetic sensors is shown in Fig. 7 and generally follow the scheme and algorithms applied in MIMU. The specific difference is the use of the output of the procedure described upper before instead of direct reading from the magnetic sensor. The result of said sensor fusion is the distance and orientation of the receiver, i.e., the second reference frame relative to the first reference frame - the transmitter.

Kinematic chain embodiment

A specific feature of the active magnetic position and orientation system is the orientation and position of the transmitter and the receiver to each other. Both reference frames associated with the transmitter 207 and solid-state magnetic sensor 210 nodes may be used as an initial reference frame. That allows a chain of nodes to be built. In such a sequence, any node serves as a reference for the next one. The theory of similar objects is known as the Kinematic chain or Linkage.

The transmitter/receiver chain is explained below:

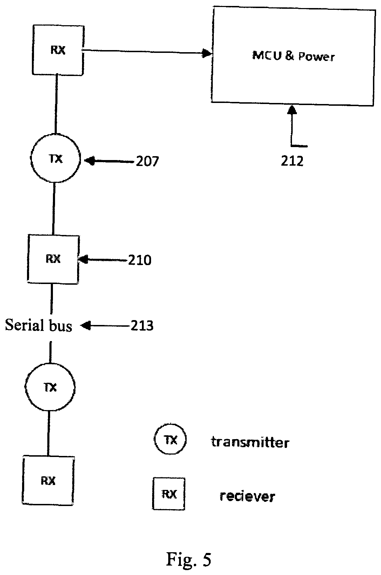

a. The distances between transmitter and receiver (solid-state magnetic sensor) are preferable to be kept smaller because of the inverse-cubed fall-off in signal with distance. In the preferred embodiment of this invention, transmitters and receivers are arranged in a way one transmitter provides a signal for two or more receivers. In the transmitter-receiver chain, as sown in fig. 5, one receiver can detect signals from two neighbour transmitters and serve as a reference frame and employ a kinematic chain.

b. The theory of kinematic chains is well known. The present invention uses some of its results to reconstruct the kinematic of the system from sensor measurements. If the position and orientation of node A 210 relative to node B 207 and the position and orientation of node B to node C 210 is known, the position and orientation of node A to node C can be calculated unambiguously.

c. The position or orientation of a three-axis magnetic source can be tracked using a corresponding three-axis magnetic sensor that detects variations in a magnetic field produced by the switchable magnetic source. For 3- dimensional position and orientation, 3 orthogonal magnetic sources are needed. In many practical applications, the motion tracking sensors are attached to body joints that have restraints. Restrains reduce the degree of freedom. For lower dimensionality (e.g., restrained movement as in cylindrical joint), one or two magnetic sources will be enough.

Data processing

Lock-in amplifier (synchronous demodulation) implemented in software is employed for improvement of signal to noise ratio without increasing the source coil's current. A lock-in amplifier (AMP) (Fig.2) provides a "baseband" output proportional to the signal under investigation. It does this by implementing synchronous demodulation of the desired signal, modulating signal source as its reference. The reference signal in the presented invention is the control signal for the H-bridge driver. The processor unit sends a request for reading for the magnetic sensor in the time frame that is designed for the reading to be performed at the moment when the transition processes in the exited coil are finished, and the magnetic field around the coil is stationary during the internal sampling of the magnetic sensor. Further, the signal output is processed by software means. The basic processing of measurements is summing the first reading and second reading multiplied by -1, i.e., vector subtraction of two consecutive measurements. The operation consists of vector subtraction of two successive measurements in the magnetic sensor resulting from the transmission of magnetic pulses in opposite direction by the magnetic transmitter. Optionally the sequence of such differences can be further analysed. The subtraction (or the adding of the first measurement to the second one multiplied by -1) has a result removal of the constant component of the magnetic field:

$$M_i = (B_i^+ + G + N_a) - (B_i^- + G + N_a)$$

Where M is the resultant magnetic vector at the moment i, $B_i^+$ and $B_i^+$ are measured magnetic vectors of pulses in opposite directions, G is the external stationary field (for example, geomagnetic), and $N_a$ is the additive noise. Generally, $B^+ = - B^-$ due to symmetry of magnetic field generated in a coil by the same current in opposite directions. Thus, the desired signal is doubled, and the undesired is zeroed, and the additive noise is decreased by a factor of 0.7 if the noise is gaussian. Further improvement of signal to noise ratio is possible by filtering the sequence Bi, and the common trade off S/N vs band. The processing may include low-pass or adaptive filtering (LPF) or usage as reference input in sensor fusion algorithm.

Embodiment for human motion capture

Preferred implementation as a motion capture system is described further as the arrangement of multiple transmitters and multiple solid-state magnetic sensors applied in close proximity as an effector/sensor chain.

The system is independent of the earth magnetic field and uses local reference. In the preferred embodiment, the solid-state magnetic sensor is used along with an inertial measurement unit. That arrangement is immune to moderate magnetic field distortion. Time-division access is used for transmitters/receivers' signal separation.

A general measurement cycle looks as follows:

    a. the processor power one of the coils in the first direction;
    b. after a programmed delay to cut out transient processes, the processor initiates the reading of the magnetic sensor;
    c. repeat steps a) and b) with coil current in inverted direction;
    d. the sequence a) to c) is repeated by other coils in the described device.

The sample sequence attained that way is filtered further. The first step is subtracting of measured magnitudes of the magnetic field in opposite directions. The described process follows the lock-in amplifier operation and remove the constant component of the magnetic field from measurement, primary geomagnetic field, and reduce additive noise. The enhancement of signal to noise ratio depends on the number of samples accumulated. Generally, a better signal to noise ratio needs more samples, and the output data rate provides.

After the measurement of the magnetic vector component by the said sensor is done, the orientation and position may be retrieved from the collected data. If a magnetic field around generating coils can be accurately mapped, it might be possible to determine the location of the sensors relative to the generating coils based on what is sensed. One to three generating coils are used depending on the application - a single angle or 3D position and orientation.

The present invention uses a mapping of the actual field around coils instead of an analytical or dipole approximation in order to optimize the efficiency of the coils.

For applications that need a higher update rate, such as motion capture, the present invention uses a fusion of orientation data obtained by the procedure with a microelectromechanical inertial sensor as described before.

A complementary Kalman filter can be applied to blend the available data from the inertial sensors and magnetic system efficiently. The inertial measurement unit provides a higher output at a rate on the order of 100 Hz. The fusion with magnetic sensor data removes the drift at the same time, providing a high angle/position update of the system as a whole.

Additionally, the local magnetic reference allows removing the translational drift. While translational drift is very significant for any inertial positioning system, for microelectromechanical IMU, it is unacceptable. For example, the orientation drift of a typical IMU is around one degree per minute since the translation drift is a tenth of meters. The local reference provided by the active magnetic system makes possible relative positioning along with relative orientation.

In the particular application of motion capture, multiple transmitters and receivers are applied in a chain with a minimum distance between magnetic source and sensor. Such arrangement reduces the power needed for coils excitation since the magnetic field strength decreases with the third power of the distance. The short path of magnetic lines also increases immunity to distortion by external ferromagnetics.

The transmitter also has an inertial sensor, who's application is for monitoring the movement of the human body. A must is to have mutual reference points (at the state of the art there is a large magnetic coil giving references for all points on the body, but since the magnetic field is rapidly decreasing, a large coil is needed). In the present invention, closely spaced sensors are used to form a chain (in the state of the art, deformation sensors are used instead of inertial).

The fusion algorithm in the present invention is modified due to the use of local reference instead of a global one, which is associated with inertial space.

Spine Monitoring Embodiment

In one embodiment of the invention, the system is used for spine monitoring sensor line. In this embodiment, a chain of transmitters and solid-state magnetic sensors as they have been defined before is applied for non-invasive monitoring of the dynamic shape of the human spine. For reliable reconstruction of the spine, measurement of kinematics by at least three angles is needed but practically a minimum of five-point coordinates are necessary. The spine curve is reconstructed by tangents in where the sensors (or markers in optic systems) are placed. In the case of absolute orientation sensors, the reconstruction algorithm used the absolute orientations and a prior knowledge of the distance between sensor points. When a local reference is used as in the present invention, the reconstruction algorithm is based on the position and orientation of the adjacent sensors relative to each other. Such algorithms are well known from the theory of kinematic chains. That information of relative position and orientation is enough for the reconstruction of the relative positions of all node's transmitters 207 and solid-state magnetic sensors 210 and consequently the construction of a smooth curve. The respective modules are connected in a daisy-chain serial bus 213, such as inter-Integrated Circuit I2C to each other and to the unit 212 including processor (MCU) and means for connecting to a power source. The present embodiment has an advantage against the absolute orientation sensor chain to measure the distances between nodes.

Hand tracking embodiment

In another embodiment of the invention, the system is used for motion tracking gloves. In this embodiment the motion tracking of fingers is challenging because of the small size and the fine motoring of the human hand. In the preferred embodiment, the palm and finger tracking system consists of a controlled unit 209 situated on top of the palm. It is comprised of processor (MCU) and power unit 212 and one transmitter 207. Five solid-state magnetic sensors 604 are placed on the second (intermediate) phalange of all five fingers. The transmitter 207 and the solid-state magnetic sensors are connected together by serial bus and are integrated into glove-like wearable. A sixth solid-state magnetic sensor 208 is situated on the lower part of the forearm and provides tracking of the wrist movement. All receivers 210 perform measurement synchronously with the transmitted signal. Receivers 210 and the transmitter 207 are equipped with magnetometers and IMU and perform sensor fusion for fast and drift-free position and orientation update. The small size of monolithic sensors makes it possible to describe tracking gloves to avoid restrictions of natural movements typical of most similar devices and to keep the power, size, and cost low.

It is also possible to use two sensors on each finger.

The low duty cycle of magnetic pulses allows the magnetic pulses of measurement sequences of different channels 801, 802 to be shifted in respect to each other in a way to exclude crosstalks and in the same time to utilize the time when the sensor has finished the sampling and is busy with measurement processing and data transfer. For this two sampling clock channels (A and B) 803 and 804, with shifted phases can be used. An example is shown in Fig. 8.

The reference numbers of the technical features are included in the claims solely for the purpose of increasing the comprehensibility of the claims and, therefore, these reference numbers do not have any limiting effect on the interpretation of the elements indicated by these reference numbers.

**Claims**

1. Active magnetic orientation and position measurement system comprising a switchable reference module including at least one solid-state magnetic sensor (210) and at least one magnetic transmitter (207), which reference module is provided with means for connecting to a power source, and a processor configured to send and receive data and commands for signal continuity from the reference module, wherein the at least one solid-state magnetic sensor (210) is configured to receive signal from the at least one magnetic transmitter (207), which comprises interconnected at least one driver circuit (201, 203, 205) and at least one transmitter coil (202, 204, 206) for generation excitation current in the form of altering DC pulses, applied to a pulse magnetic field, **characterized in that** the at least one transmitter coil (202, 204, 206) is configured to generate switching in opposite direction magnetic vectors forming a magnetic field, **and in that** the generated magnetic field is steady in switched-on position of the at least one transmitter coil (202, 204, 206), **and in that** the processor is configured to determine current position of the at least one transmitter (207) relative to the position of the at least one solid-state magnetic sensor (210), based on two subsequent measurements of said magnetic field, wherein for each pair of two subsequent measurements the first measurement is used as local reference for the second.

2. Active magnetic orientation and position measurement system according to claim 1, **characterized in that** the reference module is integrated with or connected to a micromechanical gyroscope and an accelerometer.

3. Active magnetic orientation and position measurement system according to any of the previous claims, **characterized in that** the at least one solid-state magnetic sensor (210) is a 3-axis solid-state magnetic sensor.

4. Active magnetic orientation and position measurement system according to any of the previous claims, **characterized in that** at least one inertial sensor is integrated with the at least one solid-state magnetic sensor (210).

5. Active magnetic orientation and position measurement system according to any of the previous claims, **characterized in that** it further comprises a Kalman filter.

6. Active magnetic orientation and position measurement system according to any of the previous claims, **characterized in that** it includes a chain of solid-state magnetic sensors (210) and magnetic transmitters (207) for noninvasive monitoring of the dynamic shape of a human spine, which solid-state magnetic sensors (210) and magnetic transmitters (207) are connected together by serial bus (213).

7. Active magnetic orientation and position measurement system according to any of the previous claims, **characterized in that** it includes one magnetic transmitter (207) and five solid-state magnetic sensors (210) which are connected together by serial bus and are integrated into glove-like wearable, wherein the magnetic transmitter (207) is situated on a palm of the glove-like wearable, and the five solid-state magnetic sensors (210) are situated on the intimidate phalange of all five fingers of the glove-like wearable, wherein the system comprises further a sixth solid-state magnetic sensor (210) which is situated outside the glove for tracking the movement of a human wrist.

8. Active magnetic orientation and position measurement system according to any of the previous claims, **characterized in that** the at least one driver circuit is a H-bridge driver.

9. Method for active magnetic orientation and position measurement with active magnetic orientation and position measurement system according to claims 1-8, comprising the steps of:

- providing DC current in a result of a command from a processor to at least one transmitter coil (202, 204, 206) from a reference module in a first direction;
- after a programmed in the processor delay to cut out transient processes, reading from a processor data received from at least one solid-state magnetic sensor (210) from the reference module regarding the orientation and position in the first direction;
- providing DC current in a result of a command from the processor to the at least one transmitter coil (202, 204, 206) from the reference module in a second opposite to the first direction;
- after a programmed delay in the processor to cut out transient processes, reading from a processor data received from the at least one solid-state magnetic sensor (210) regarding the orientation and position in the second opposite to the first direction, and determining a current position of the at least one solid-state magnetic sensor (210);
- generating new DC current pulses to the at least one transmitter coil (202, 204, 206) for a subsequent measurement from the processor of data regarding the orientation and position in the first and second direction;
- using the first measured values from the generated magnetic field as a local reference to determine a new current orientation and position of the at least one solid-state magnetic sensor (210).

10. Method for active magnetic orientation and position measurement according to claim 9, **characterized in that** the reference module includes more than one transmitter (207) and solid-state magnetic sensors (210), which orientations and positions are also measured in subsequent periods based on the measurement of the generated magnetic vectors in two opposite directions.

Fig. 1

Fig. 2

Fig. 3

Fig. 5

Fig. 6

Fig. 7

Fig. 8

Europäisches
Patentamt

European
Patent Office

Office européen
des brevets

# EUROPEAN SEARCH REPORT

**Application Number**

EP 24 47 2007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2020/272235 A1 (NG SHIU SANG [US]) 27 August 2020 (2020-08-27) <br> * figures 2, 5 * <br> * paragraphs [0004] - [0006], [0014] * <br> * paragraphs [0027], [0058] - [0062] * <br> - - - - - | 1-10 | INV. <br> A61B5/00 <br> A61B5/06 <br> G01B7/004 <br> G06F3/01 |
| Y | US 2006/293593 A1 (GOVARI ASSAF [IL] ET AL) 28 December 2006 (2006-12-28) <br> * figure 4 * <br> * paragraphs [0006] - [0009] * <br> * paragraphs [0050] - [0059] * <br> - - - - - | 1-10 | |
| A | CN 108 030 495 B (SHENZHEN NAFUTE TECH CO LTD) 4 September 2020 (2020-09-04) <br> * paragraphs [0020] - [0031], [0039] * <br> - - - - - | 1-10 | |
| A | WIELGOS SAM ET AL: "Garment Integrated Spinal Posture Detection Using Wearable Magnetic Sensors", <br> 2020 42ND ANNUAL INTERNATIONAL CONFERENCE OF THE IEEE ENGINEERING IN MEDICINE & BIOLOGY SOCIETY (EMBC), IEEE, <br> 20 July 2020 (2020-07-20), pages 4030-4033, XP033815701, <br> DOI: 10.1109/EMBC44109.2020.9175725 <br> [retrieved on 2020-08-24] <br> * abstract * <br> * figure 1 * <br> - - - - - | 1-10 | **TECHNICAL FIELDS SEARCHED (IPC)** <br><br> A61B <br> G01B <br> G06F |

The present search report has been drawn up for all claims

| Place of search | Date of completion of the search | Examiner |
|---|---|---|
| The Hague | 18 July 2024 | Athanasiadis, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 47 2007

18-07-2024

| Patent document cited in search report | Publication date | Patent family member(s) | Publication date |
|---|---|---|---|
| US 2020272235 A1 | 27-08-2020 | CN 111596756 A | 28-08-2020 |
|  |  | US 2020272235 A1 | 27-08-2020 |
| US 2006293593 A1 | 28-12-2006 | AT E500794 T1 | 15-03-2011 |
|  |  | AU 2006202054 A1 | 30-11-2006 |
|  |  | AU 2010224433 A1 | 21-10-2010 |
|  |  | BR PI0601783 A | 09-01-2007 |
|  |  | CA 2546883 A1 | 16-11-2006 |
|  |  | CN 1868403 A | 29-11-2006 |
|  |  | DK 1723923 T3 | 23-11-2009 |
|  |  | EP 1723923 A2 | 22-11-2006 |
|  |  | EP 2039315 A1 | 25-03-2009 |
|  |  | ES 2327866 T3 | 04-11-2009 |
|  |  | HK 1098319 A1 | 20-07-2007 |
|  |  | HK 1129553 A1 | 04-12-2009 |
|  |  | IL 175648 A | 30-11-2010 |
|  |  | JP 5068476 B2 | 07-11-2012 |
|  |  | JP 2006346443 A | 28-12-2006 |
|  |  | KR 20060118360 A | 23-11-2006 |
|  |  | US 2006293593 A1 | 28-12-2006 |
| CN 108030495 B | 04-09-2020 | NONE |  |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 5307072 A